(19)

**Europäisches Patentamt**
**European Patent Office**
**Office européen des brevets**

(11) **EP 4 224 193 A1**

(12) **EUROPEAN PATENT APPLICATION**

(43) Date of publication:
**09.08.2023 Bulletin 2023/32**

(21) Application number: **22156238.2**

(22) Date of filing: **11.02.2022**

(51) International Patent Classification (IPC):
**G01R 33/54** (2006.01)   **G01R 33/561** (2006.01)
**A61B 5/055** (2006.01)   **G01R 33/56** (2006.01)
**G06N 3/02** (2006.01)   **G01R 33/48** (2006.01)

(52) Cooperative Patent Classification (CPC):
**G01R 33/561; G01R 33/543;** A61B 5/055;
G01R 33/4818; G01R 33/5608; G06N 3/02

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.02.2022 US 202263306526 P**

(71) Applicant: **Koninklijke Philips N.V.**
**5656 AG Eindhoven (NL)**

(72) Inventors:
• **AMTHOR, Thomas Erik**
  **Eindhoven (NL)**
• **BOERNERT, Peter**
  **Eindhoven (NL)**
• **SCHUELKE, Christophe Michael Jean**
  **Eindhoven (NL)**
• **DUENSING, George Randall**
  **Eindhoven (NL)**

(74) Representative: **Philips Intellectual Property &
Standards**
**High Tech Campus 52**
**5656 AG Eindhoven (NL)**

(54) **AUTOMATIC OPTIMIZATION OF MR EXAMINATION PROTOCOLS**

(57)    The invention relates to a method for optimizing an examination protocol for executing a magnetic resonance (MR) image acquisition from a body of a patient. It is an object of the invention to facilitate efficient implementation of accelerated (e.g., artificial intelligence-based) examination protocols that are a true or very close replacement for examination protocols already existing in clinical practice. It should be made possible to provide each individual clinic with specific optimized versions of their own standard examination protocols. As a solution, the method of the invention comprises the steps of: providing an examination protocol containing specifications of two or more imaging sequences; in a computer, executing at least one algorithm processing said examination protocol as an input to perform an optimization with regard to the speed of execution of the examination protocol, taking into account diagnostic relevance weightings assigned to the imaging sequences contained in the examination protocol; and making an output available representing said optimized examination protocol to a user and/or executing the MR image acquisition on an MR scanner based on said optimized examination protocol. Moreover, the invention relates to an MR scanner (1), to a computer (15) and to a computer program for an MR scanner (1).

Fig. 2

EP 4 224 193 A1

**Description**

FIELD OF THE INVENTION

The present invention relates to the field of magnetic resonance (MR). It finds particular application in conjunction with MR imaging methods and MR scanners for diagnostic purposes, and will be described with particular reference thereto.

BACKGROUND OF THE INVENTION

Image-forming MR methods which utilize the interaction between magnetic fields and nuclear spins in order to form two-dimensional or three-dimensional images are widely used nowadays, notably in the field of medical diagnostics, because for the imaging of soft tissue they are superior to other imaging methods in many respects, do not require ionizing radiation and are usually not invasive.

In fact, MR imaging is often the most sensitive or appropriate technique in clinical diagnosis, but lengthy acquisition times limit its use due to cost and considerations of patient comfort and compliance. Once an image field of view and resolution is chosen, the minimum MR image acquisition time is normally determined by the amount of raw data that must be acquired to meet the Nyquist criteria.

The expected post-COVID surge of patients in radiologic departments clearly shows the need for highly accelerated imaging procedures and reduction of examination protocols to the essential features in situations where there is an unusually high patient volume. Similar situations arise when the number of cases to be examined rises when imaging systems have not been operational over a period of time for whatever reasons.

A major development in accelerated MR acquisition during the past years was parallel imaging (PI). With PI techniques, the spatial sensitivity profiles of multiple receiving coils of the MR scanner used provide additional information for the reconstruction, allowing for successful reconstruction from undersampled k-space data. Two common PI methods are generalized autocalibrating partial parallel acquisition (GRAPPA), and sensitivity encoding (SENSE). A particularly successful method for accelerating MR image acquisition that uses a sparsity prior and incoherent sampling is compressed sensing (CS). CS reconstruction is an iterative reconstruction method, which estimates the MR image from (strongly) undersampled MR signal data by enforcing data consistency and utilizing the assumption that medical images are compressible (ie, sparse in some spatial transform domain). A drawback of these known approaches of accelerated acquisition is that the reconstruction problems that PI and CS methods are designed to solve are themselves time-consuming. While reconstruction can be done offline, clinical scenarios require speed not only for the actual MR signal acquisition but also for the reconstruction of individual scans.

On the other hand, artificial intelligence (AI)-assisted acceleration methods for MR imaging are quickly emerging (see review by Lin et al., J. Magn. Reson. Imaging 2021, 53:1015-1028), opening up ways to greatly reduce scan time (by allowing for successful reconstruction from strongly undersampled k-space data) while maintaining image quality to a large extent. AI-based image acquisition and reconstruction techniques, in particular those based on machine learning (or deep learning) are useful because they perform the optimization over many training images prior to solving the reconstruction for any particular given image. They can take advantage of common features of anatomy as well as the structure of undersampling artifacts that are present across the training images. Thus, for a new scan, unlike CS reconstructions, which each require lengthy computation time, deep learning-based reconstruction models can complete the reconstruction in seconds. It is for these reasons that a lot of research is presently investigated into the use of deep learning-based approaches to achieve accelerated, high-quality MR image reconstruction.

However, implementation of these AI methods is difficult and usually requires large training data sets in order to be generalizable. Furthermore, AI-based image reconstruction can only be used for image contrasts that the respective reconstruction model has been trained on, which in general does not match exactly what radiologists are used to seeing from their individual standard examination protocols and imaging sequence definitions. In a situation where examinations need to be sped up immediately, clinics cannot simply replace their standard examination protocols with improved and accelerated AI-based technology (if available at all), because the generated image contrasts depend on the trained AI models and in general do not match exactly the appearance of the standard protocols used in their daily work. Often each site has some most favorite scans and procedures which vary among clinics although standardization is often desired.

SUMMARY OF THE INVENTION

From the foregoing it is readily appreciated that there is a need for an improved method of optimizing MR examination protocols in terms of acquisition speed. It is consequently an object of the invention to facilitate efficient implementation of accelerated examination protocols that are a true or very close replacement for examination protocols already existing in clinical practice. It should be made possible to provide each individual clinic with specific optimized versions of their own standard examination protocols.

In accordance with the invention, a method for optimizing an examination protocol for executing an MR image acquisition from a body of a patient is disclosed. The method comprises the following steps:

- providing an examination protocol containing specifications of two or more imaging sequences;
- in a computer, executing at least one algorithm processing said examination protocol as an input to perform an optimization with regard to the speed of execution of the examination protocol, taking into account diagnostic relevance weightings assigned to the imaging sequences contained in the examination protocol; and
- making an output available representing said optimized examination protocol to a user and/or executing the MR image acquisition on an MR scanner based on said optimized examination protocol.

The proposed method automatically assesses and exploits the optimization potential of arbitrary examination protocols. The invention thus achieves acceleration for exactly the individually required examination protocol. For each imaging sequence contained in in the examination protocol, a clinician (e.g., a radiologist) specifies the diagnostic relevance as a weighting factor (e.g., as $0 \leq w_i \leq 1$) for use by the algorithm in the optimization method. The diagnostic relevance represents the relative importance of obtaining a good image quality from the respective individual imaging sequence for a specific diagnostic purpose. For example, if knowledge of the physiology of the examined organ of the patient is more important than anatomic aspects, then a clinician may decide that the image quality of the imaging sequence providing a diffusion-weighted contrast and a $T_2$-weighted contrast in the examination protocol should have higher relevance than that of an imaging sequence providing a $T_1$-weighted contrast scan in the same examination protocol. By defining the diagnostic relevance weightings it can be taken into account that it may not only by the individual scan or contrast that is of importance but also the combination (in the sense the combination is often more than the sum of the individuals).

The result of the optimization, i.e. the accelerated examination protocol is finally made available, e.g. to the clinician, for further use. The user may be notified of the result of the automatic optimization e.g. by a graphical representation of the generated output examination protocol on a display monitor. The notification typically takes the form of a suggestion to the user. A final determination regarding which examination protocol will be executed is made by the user. The user may apply modifications to the proposed examination protocol based on his individual knowledge and experience before he initiates the actual MR image acquisition.

The examination protocol may comprise different acquisition parameters specifying the two or more imaging sequences. Such parameters will mainly depend on the respective clinical question. The parameters can determine different physical variables, such as a voltage to be applied to a component, for example a radio-frequency antenna of the MR scanner used. A parameter can also be a duration between two radio-frequency pulses and/or magnetic field gradients to be activated. By stipulating a resolution, for example, the parameters can characterize the MR image to be acquired. Further parameters can determine the location, orientation and size of the field of view as well as the contrast-weighting (e.g. $T_1$-weighting, $T_2$-weighting, diffusion-weighting etc.) of the MR image acquisition. In an embodiment of the invention, the optimization involves a modification of the acquisition parameters associated with at least one of the imaging sequences so as to accelerate the execution of the imaging sequence. Similarly, the optimization may involve a modification of the k-space sampling pattern and/or of the image reconstruction model associated with at least one of the imaging sequences. E.g., the algorithm may replace a given imaging sequence by a PI or CS pendant. This obviously requires not only a modification of the k-space sampling pattern but also of the image reconstruction model (i.e., a detailed and complete definition of the reconstruction procedure associated with the specific optimized imaging sequence) required to compute a high-quality MR image from the undersampled k-space data. To this end, the invention proposes the concept of a model-enhanced optimized examination protocol which does not only specify the accelerated imaging sequences to be executed but also adds the corresponding reconstruction model to the output made available by the algorithm. When provided with such a model-enhanced examination protocol, the MR reconstruction engine used in the execution of the MR examination based on the optimized examination protocol has all the information necessary to reconstruct the MR images from the acquired MR signal data.

Similarly, the algorithm may replace a given imaging sequence by an AI pendant, wherein the optimization may involve assigning the associated AI-based image reconstruction model to the respective imaging sequences. The AI-based image reconstruction model may use a machine learning method, wherein the optimization involves training the AI-based image reconstruction model and incorporating the trained artificial intelligence-based image reconstruction model into the output to provide a corresponding "ready-to-use" model-enhanced examination protocol as explained above. Selecting AI-based image acquisition and reconstruction for a specific imaging protocol depends on the exact type of imaging sequence definition and desired image contrast, as well as the availability of suitable training data (e.g., example MR images acquired with the original examination protocol). It is an insight of the invention that individually designed and trained AI reconstruction models perform best when applied with the specific imaging sequence they were trained with. Consequently, the trained AI reconstruction model should not be a fixed part of the reconstruction engine used for the MR examination. Instead, the trained AI reconstruction model should be provided as an output of the protocol optimization together with the individual optimized imaging sequence definition. While a standard examination protocol only contains acquisition instructions for the contained imaging sequences, the model-enhanced examination protocol of the invention also contains the AI reconstruction model trained for reconstruction with the specific imaging sequences, ideally along with a set of detailed instructions specifying how the reconstruction model should be used.

In another embodiment, the optimization may involve assigning an image reconstruction model to at least one of the imaging sequences which image reconstruction model uses MR signal data acquired by executing at least one of the other imaging sequences. This means that, for the purpose of acceleration of the MR image acquisition, the reconstruction of MR images associated with some of the imaging sequences contained in the input examination protocol may be based on MR signal data acquired by other imaging sequences. The introduction of such sharing of MR signal data between the different imaging sequences can be part of the optimization according to the invention. Possibly, the execution of at least one of the imaging sequences is entirely omitted in the optimized examination protocol, wherein an image reconstruction model is added to the optimized examination protocol to synthesize an MR image associated with the omitted imaging sequence from MR signal data acquired by executing a least one of the other imaging sequences. If the optimization algorithm finds out that it is not necessary to execute every imaging sequence contained in the examination protocol but to synthesize the MR images associated with one or more of the imaging sequences from the MR signal data acquired by one or more of the other imaging sequences the algorithm can decide to omit the execution of the respective imaging sequence resulting in a correspondingly significant increase of the speed of execution of the examination protocol. For example, a suitable reconstruction model can synthesize an estimate of a $T_2$-weighted MR image from a survey image and a $T_1$-weighted MR image. Such way of optimization of the examination protocol may make it necessary to also modify the order in which the imaging sequences are executed, because the reconstruction of MR images from some (optimized) imaging sequences may be based on the MR images from previous imaging sequences in the examination protocol.

In another embodiment, the at least one algorithm further takes a quality trade-off weight into account which is a user-specified trade-off between quality of the MR images resulting from the optimized examination protocol and the increase of execution speed achieved by the optimization. This can be expressed as a further weighting factor $0 \le p \le 1$, where larger values of $p$ result in higher acceleration with more compromises regarding image quality. If more than one optimized version of the examination protocol is desired (e.g., for different acceleration stages), a list of several weighting factors can be specified, each one resulting in the generation of one optimized examination protocol. In a practical realization, the algorithm may determine a number of optimization options and select one of the optimization options in accordance with an objective function that assigns a higher weighting to an optimization option which results in a higher image quality of those MR images associated with imaging sequences having higher diagnostic relevance weightings and simultaneously in a lower image quality of those MR images associated with imaging sequences having lower diagnostic relevance weightings.

The method of the invention described thus far can be carried out by means of a computer used in a clinical workflow. The computer may be a control computer of an MR scanner including at least one main magnet coil for generating a main magnetic field within an examination volume, a number of gradient coils for generating switched magnetic field gradients in different spatial directions within the examination volume, at least one RF coil for generating RF pulses within the examination volume and/or for receiving MR signals from a body of a patient positioned in the examination volume, with the control computer controlling the temporal succession of RF pulses and switched magnetic field gradients based on an examination protocol, and a reconstruction unit for reconstructing MR images from the received MR signals. The method of the invention can be implemented by a corresponding programming/software of the control computer of the MR scanner.

The method of the invention can be advantageously carried out in most MR environments in clinical use at present. To this end it is merely necessary to utilize a computer program by which the computer to be used is controlled such that it performs the above-explained method steps of the invention. The computer program may be present either on a data carrier or be present in a data network so as to be downloaded for installation in the computer, e.g. the control computer of an MR scanner.

## BRIEF DESCRIPTION OF THE DRAWINGS

The enclosed drawings disclose preferred embodiments of the present invention. It should be understood, however, that the drawings are designed for the purpose of illustration only and not as a definition of the limits of the invention. In the drawings:

Fig. 1 shows an MR scanner for carrying out the method of the invention;

Fig. 2 illustrates an embodiment of the method of the invention as a flow chart;

Fig. 3 schematically illustrates the concept of a model-enhanced examination protocol in accordance with the invention.

## DETAILED DESCRIPTION OF THE EMBODIMENTS

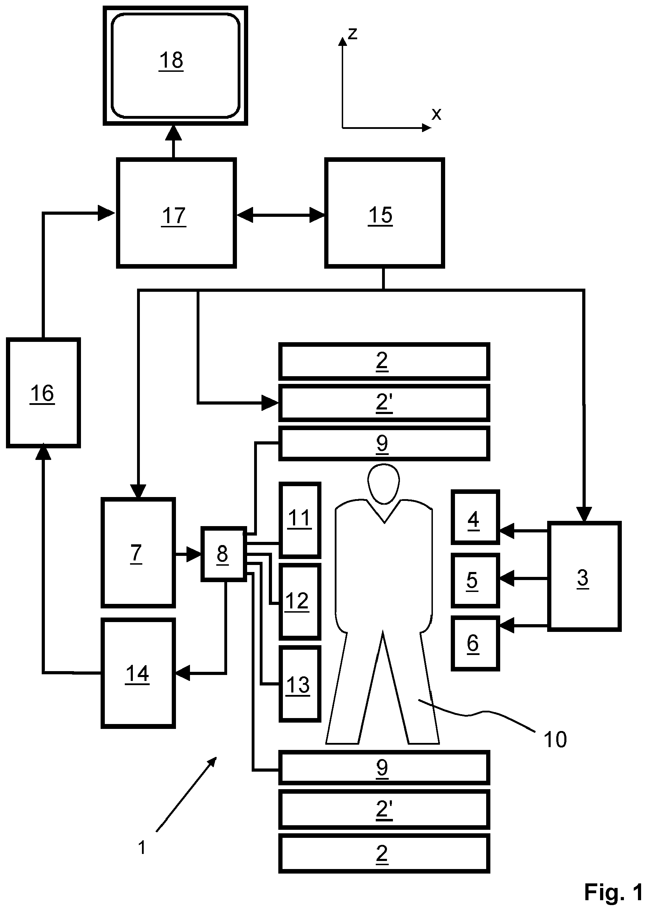

With reference to Fig. 1, an MR scanner 1 is shown. The scanner comprises superconducting or resistive main magnet coils 2 such that a substantially uniform, temporally constant main magnetic field $B_0$ is cre-

ated along a z-axis through an examination volume. The device further comprises a set of (1st, 2nd, and - where applicable - 3rd order) shimming coils 2', wherein the current flow through the individual shimming coils of the set 2' is controllable for the purpose of minimizing $B_0$ deviations within the examination volume.

A magnetic resonance generation and manipulation system applies a series of RF pulses and switched magnetic field gradients to invert or excite nuclear magnetic spins, induce magnetic resonance, refocus magnetic resonance, manipulate magnetic resonance, spatially and otherwise encode the magnetic resonance, saturate spins, and the like to perform MR imaging.

Most specifically, a gradient pulse amplifier 3 applies current pulses to selected ones of whole-body gradient coils 4, 5 and 6 along x, y and z-axes of the examination volume. A digital RF frequency transmitter 7 transmits RF pulses or pulse packets, via a send-/receive switch 8, to a body RF coil 9 to transmit RF pulses into the examination volume. A typical MR pulse sequence is composed of a packet of RF pulse segments of short duration which taken together with each other and any applied magnetic field gradients achieve a selected manipulation of nuclear magnetic resonance. The RF pulses are used to saturate, excite resonance, invert magnetization, refocus resonance, or manipulate resonance and select a portion of a body 10 positioned in the examination volume. The MR signals are also picked up by the body RF coil 9.

For generation of MR images of limited regions of the body 10 by means of parallel imaging, a set of local array RF coils 11, 12, 13 are placed contiguous to the region selected for imaging. The array coils 11, 12, 13 can be used to receive MR signals induced by body-coil RF transmissions.

The resultant MR signals are picked up by the body RF coil 9 and/or by the array RF coils 11, 12, 13 and demodulated by a receiver 14 preferably including a preamplifier (not shown). The receiver 14 is connected to the RF coils 9, 11, 12 and 13 via send-/receive switch 8.

A control computer 15 controls the current flow through the shimming coils 2' as well as the gradient pulse amplifier 3 and the transmitter 7 to generate any of a plurality of MR pulse sequences, such as echo planar imaging (EPI), echo volume imaging, gradient and spin echo imaging, fast spin echo imaging, and the like in conformity with a pre-determined examination protocol. For the respective examination protocol, the receiver 14 receives a single or a plurality of MR data lines in rapid succession following each RF excitation pulse. A data acquisition system 16 performs analog-to-digital conversion of the received signals and converts each MR data line to a digital format suitable for further processing. In modern MR scanners the data acquisition system 16 is a separate computer which is specialized in acquisition of raw image data. The examination protocol determines the imaging sequences, i.e. the MR pulse sequences including the frequency and the temporal succession of radio-frequency pulses and/or magnetic field gradients to be activated. The parameters included in the examination protocol thereby characterize the MR image to be acquired, in particular with regard to location, orientation and size of the field of view as well as the contrast-weighting.

Ultimately, the digital raw image data is reconstructed into an image representation by a reconstruction processor 17 which applies a Fourier transform or other appropriate reconstruction algorithms, such like SENSE or SMASH. The MR image may represent a planar slice through the patient, an array of parallel planar slices, a three-dimensional volume, or the like. The image is then stored in an image memory where it may be accessed for converting slices, projections, or other portions of the image representation into appropriate format for visualization, for example via a video monitor 18 which provides a man-readable display of the resultant MR image.

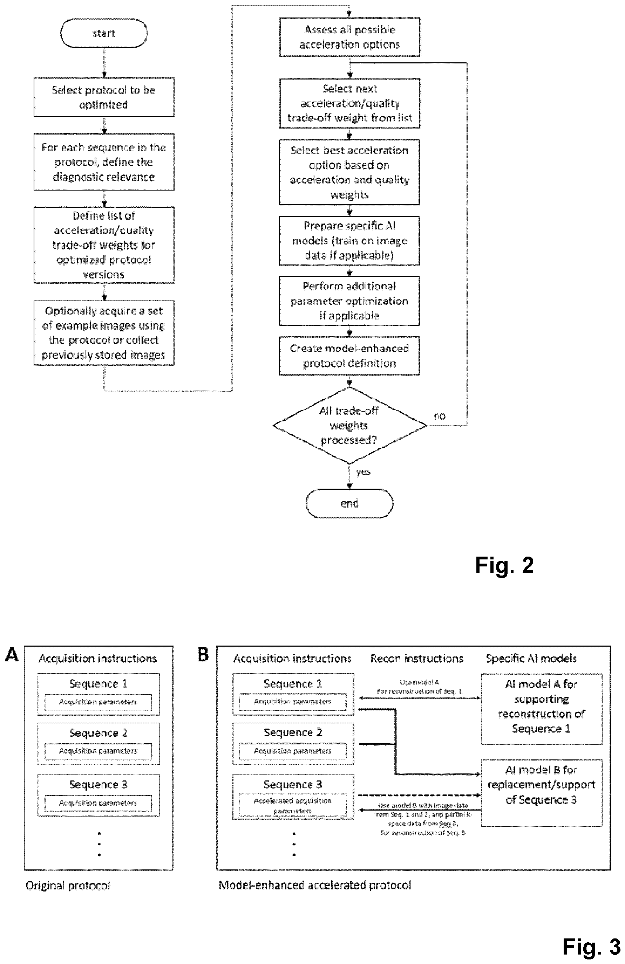

According to Fig. 2, the method of the invention starts with the selection of an examination protocol to be optimized. The examination protocol contains specifications and parameters of two or more imaging sequences.

For each imaging sequence contained in in the examination protocol, a clinician (e.g., a radiologist) defines a diagnostic relevance as a weighting factor $0 \leq w_i \leq 1$. The diagnostic relevance represents the relative importance of obtaining a good image quality from the respective individual imaging sequence for a specific diagnostic purpose.

In the next step, the clinician defines a list of quality trade-off weights for the optimization process. Each of these specifies a trade-off between quality of the MR images resulting from the optimized examination protocol and the increase of execution speed achieved by the optimization. This can be expressed as a further weighting factor $0 \leq p \leq 1$, where larger values of $p$ result in higher acceleration with more compromises regarding image quality. In the depicted embodiment, more than one optimized version of the selected examination protocol is desired (e.g., for assessing different acceleration stages) such that a list of several weighting factors is specified, each one resulting in the generation of one optimized examination protocol.

Optionally a set of example MR images is acquired using the examination protocol to be optimized. Alternatively, previously stored images may be collected. These images can be used later on in the optimization process for training an AI reconstruction model.

As a next step, the examination protocol with the diagnostic relevance weights, the trade-off weights and the example image data are provided as an input to an algorithm running on a computer to perform an optimization with regard to the speed of execution of the examination protocol. The algorithm assesses a number of acceleration options. Different acceleration techniques, some AI-based, are employed to accelerate the examination protocol. The types of acceleration techniques feasible for a specific examination protocol depend on the exact type of the imaging sequences and image contrasts, as well as the availability of training data (e.g., example images acquired with the original protocol). Three main classes of acceleration techniques can be distinguished:

AI-based image reconstruction:
The reconstruction of one or more images from the MR signal data acquired by one or more of the imaging sequences contained in the examination protocol is supported by a trained AI reconstruction model, for example by calculating diagnostic-quality images from a heavily undersampled k-space data set.
AI-based generation of additional contrasts:
A trained AI reconstruction model synthesizes an MR image associated to one of the imaging sequences based on MR signal data or image of other imaging sequences, thereby replacing or reducing the amount of MR signal data to be acquired. These synthetic contrasts can be used directly for diagnosis, if the confidence that the synthetic images are correct is very high. Alternatively, they can serve as prior knowledge in the reconstruction of a highly undersampled acquisition of the specific contrast.

Tuning of acquisition parameters and/or applying PI- or CS-based techniques without the use of any AI-enhanced image reconstruction.

An acceleration option can be regarded as a specific set of acceleration techniques that can be applied to the examination protocol to be optimized. By evaluating all possible combinations of acceleration techniques for the protocol, a set of acceleration options is proposed.

The best out of $M$ acceleration options is chosen according to an objective function including weights for the acceleration and quality aspects. A preferred embodiment of such an objective function is described in the following.

Let $N$ be the number of imaging sequences in the examination protocol with $d_i$ being the scan duration of sequence $i$.

The acceleration factor $A_k$ of acceleration option $k$ is defined as the ratio of the total scan duration without optimizations and the total scan duration of option $k$, where $\hat{d}_{k,i}$ denotes the duration of scan $i$ in acceleration option $k$:

$$A_k = \sum_{i=1}^{N} d_i \Big/ \sum_{i=1}^{N} \hat{d}_{k,i}$$

The relative quality estimators $0 \leq q_{k,i} \leq 1$ represent the expected diagnostic image quality of the accelerated imaging sequence $i$ of acceleration option $k$ relative to the image quality of the original imaging sequence. There are objective quality criteria that can be attributed to the obtained images in relation to the images obtained by the original sequences such that it is possible to determine the relative quality estimators entirely automatically. However, in a number of practical cases the quality values may depend to some degree on the subjective impression of a radiologist. A possible implementation can thus be a prior determination of relative quality estimators for a number of acceleration approaches in an empirical way, i.e. by requesting feedback from one or more radiologists. The relative quality estimators obtained are stored in a table, so that for each acceleration approach and each relevant context information (e.g., anatomy, clinical question), the corresponding relative quality estimator can be looked up.

The quality factor $Q_k$ of acceleration option $k$ is defined as the weighted sum of the relative quality estimators $q_{k,i}$, where the weighting factors $0 \leq w_i \leq 1$ described above depend on the application of the protocol and represent the relevance of image quality for each sequence $i$:

$$Q_k = \sum_{i=1}^{N} w_i \ q_{k,i}$$

The acceleration trade-off weight $0 \leq p \leq 1$ specifies the overall importance of acceleration vs. image quality. Larger values of $p$ result in higher acceleration with more compromises in image quality.

To determine the best acceleration option, the overall objective function

$$f_k = p A_k + (1-p) Q_k$$

is maximized to find $k_{\text{opt}}$:

$$k_{opt} = \operatorname*{argmax}_{k} f_k$$

The optimization is performed for multiple values of $p$, resulting in multiple versions of the accelerated examination protocol with different acceleration factors. The clinician can then decide which version to choose, in order to optimally fill the available time slot for the examination.

Once an acceleration option has been selected, the required AI-based reconstruction models are computed by training on either the supplied example images or synthetically generated data. Pre-trained AI reconstruction models may be available that are then only retrained and adapted to the specific type of images under consideration according to the examination protocol.

It is an insight of the invention, that individually designed and trained AI-based reconstruction models perform best when applied on the MR signal data acquired by executing the specific imaging sequence they were trained with. Consequently, the trained AI-based reconstruction models should be provided as an output of the above-described optimization procedure together with the individual optimized imaging sequence definition. To this end, the invention proposes the concept of a model-enhanced imaging protocol definition (or model-enhanced "ExamCard") as shown in Fig. 3. While the standard (conventional) examination protocol (protocol A in Figure 3) only contains acquisition instructions for the different imaging sequences defined by the respective acquisition parameters, the model-enhanced examination protocol (protocol B in Fig. 3) also contains the AI-based reconstruction models trained for reconstruction of MR images from MR signal data acquired by executing the specific imaging sequences along with an instruction set ("Recon instructions" in Fig. 2) specifying how the reconstructions models should be applied and which MR signal data serve as input. When provided with such a model-enhanced examination protocol, the MR reconstruction engine (reconstruction processor 17 in Fig. 1) has all the information available to reconstruct the images in the specifically designed, optimized way. In the shown embodiment, the reconstruction of MR images from the MR signal data of some of the imaging sequences is based on the MR signal data and/or image data resulting from other imaging sequences in the embodiment of Fig. 3. As a result, the order of execution of the imaging sequences becomes important, and the optimized examination protocol may have a different, optimized sequence order than the original examination protocol. In the embodiment shown, AI-based reconstruction model A is used for reconstructing an MR image from the MR signal data resulting from the execution of imaging sequence 1. The image data from imaging sequences 1 and 2 is used by AI-based reconstruction model B for replacement or for support of imaging sequence 3. E.g., AI model B uses image data from sequence 1 and sequence 2 and partial k-space data from sequence 3 for reconstructing/synthesizing an MR image of the contrast associated with sequence 3.

In a possible embodiment, the invention may be applied as an optimization service offered by a service provider (e.g. a manufacturer of MR scanners). Customers may send their own examination protocols (e.g., "ExamCards"), their diagnostic preferences (diagnostic relevance weights), and ideally a set of example images to the optimization service. The examination protocol is then optimized by a computer run by the optimization service for increasing acquisition speed following the method of the invention as described above. The customer receives one or several optimized model-enhanced versions of the specific examination protocol, suitable for direct "ready-to-use" replacement of the original examination protocol.

## Claims

1. Method for optimizing an examination protocol for executing an MR image acquisition from a body of a patient, the method comprising the following steps:

   - providing an examination protocol containing specifications of two or more imaging sequences;
   - in a computer, executing at least one algorithm processing said examination protocol as an input to perform an optimization with regard to the speed of execution of the examination protocol, taking into account diagnostic relevance weightings assigned to the imaging sequences contained in the examination protocol; and
   - making an output available representing said optimized examination protocol to a user and/or executing the MR image acquisition on an MR scanner based on said optimized examination protocol.

2. Method of claim 1, wherein the optimization involves a modification of acquisition parameters associated with at least one of the imaging sequences.

3. Method of claim 1 or 2, wherein the optimization involves a modification of the k-space sampling pattern and/or of the image reconstruction model associated with at least one of the imaging sequences.

4. Method of claim 3, wherein the k-space sampling pattern is modified to result in an undersampling of k-space.

5. Method of any one of claims 1-4, wherein the optimization involves assigning an artificial intelligence-based image reconstruction model to at least one of the imaging sequences.

6. Method of claim 5, wherein the artificial intelligence-based image reconstruction model uses a machine learning method, wherein the optimization involves training the artificial intelligence-based image reconstruction model and incorporating the trained artificial intelligence-based image reconstruction model into the output.

7. Method of any one of claims 1-6, wherein the optimization involves assigning an image reconstruction model to at least one of the imaging sequences which image reconstruction model uses MR signal data or image data acquired by executing at least one of the other imaging sequences.

8. Method of any one of claims 1-7, wherein the execution of at least one of the imaging sequences is omitted in the optimized examination protocol, wherein an image reconstruction model is added to the optimized examination protocol to synthesize an MR image associated with the omitted imaging sequence from MR signal data acquired by executing a least one of the other imaging sequences.

9. Method of any one of claims 1-8, wherein the optimization involves a modification of the order in which the imaging sequences contained in the examination protocol are executed.

10. Method of any one of claims 1-9, wherein the at least one algorithm further takes a quality trade-off weight into account which is a user-specified trade-off between quality of the MR images resulting from the optimized examination protocol and the increase of execution speed achieved by the optimization.

11. Method of any one of claims 1-10, wherein the algorithm determines a number of optimization options and selects one of the optimization options in accordance with an objective function that assigns a higher weighting to an optimization option which results in a higher image quality of those MR images associated with imaging sequences having higher diagnostic relevance weightings and simultaneously in a lower image quality of those MR images associated with imaging sequences having lower diagnostic relevance weightings.

12. Computer, configured to:

- read a digital representation of an examination protocol containing specifications of two or more imaging sequences;
- execute at least one algorithm processing said examination protocol as an input to perform an optimization with regard to the speed of execution of the examination protocol, taking into account diagnostic relevance weightings assigned to the imaging sequences contained in the examination protocol; and
- make an output available representing said optimized examination protocol.

13. Computer program comprising instructions for:

- reading a digital representation of an examination protocol containing specifications of two or more imaging sequences;
- executing at least one algorithm processing said examination protocol as an input to perform an optimization with regard to the speed of execution of the examination protocol, taking into account diagnostic relevance weightings assigned to the imaging sequences contained in the examination protocol; and
- making an output available representing said optimized examination protocol.

14. MR scanner comprising at least one main magnet coil (2) for generating a main magnetic field within an examination volume, a number of gradient coils (4, 5, 6) for generating switched magnetic field gradients in different spatial directions within the examination volume, at least one RF coil (9) for generating RF pulses within the examination volume and/or for receiving MR signals from a body (10) of a patient positioned in the examination volume, a control computer (15) for controlling a temporal succession of RF pulses and switched magnetic field gradients based on an examination protocol (32), and a reconstruction unit (17) for reconstructing MR images (33) from the received MR signals, wherein the MR scanner (1) is arranged to perform the following steps:

- reading a digital representation of the examination protocol containing specifications of two or more imaging sequences into the control computer (15);
- in the control computer (15), executing at least one algorithm processing said examination protocol as an input to perform an optimization with regard to the speed of execution of the examination protocol, taking into account diagnostic relevance weightings assigned to the imaging sequences contained in the examination protocol; and
- executing an MR image acquisition (28) on the MR scanner (1) based on said optimized examination protocol (32).

Fig. 1

**Fig. 2**

**Fig. 3**

Europäisches
Patentamt

European
Patent Office

Office européen
des brevets

**EUROPEAN SEARCH REPORT**

Application Number

EP 22 15 6238

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/197841 A1 (FLEYSHER LAZAR [US] ET AL) 21 August 2008 (2008-08-21) | 1,2,5-7, 10,12-14 | INV. G01R33/54 |
| A | * paragraph [0018] - paragraph [0019]; figures 1a,1b * <br> * paragraph [0043] - paragraph [0054] * <br> * paragraph [0076] - paragraph [0078] * <br> * paragraphs [0093], [0133] and [0135] * | 3,4,8,9, 11 | G01R33/561 <br><br> ADD. A61B5/055 G01R33/56 G06N3/02 |
| X | SOLTANIAN-ZADEH H ET AL: "OPTIMIZATION OF MRI PROTOCOLS AND PULSE SEQUENCE PARAMETERS FOR EIGENIMAGE FILTERING", IEEE TRANSACTIONS ON MEDICAL IMAGING, IEEE, USA, vol. 13, no. 1, 1 March 1994 (1994-03-01), pages 161-175, XP000440160, ISSN: 0278-0062, DOI: 10.1109/42.276155 | 1,2,5-7, 10,12-14 | G01R33/48 |
| A | * abstract, sections I, II.A and III.A * | 3,4,8,9, 11 | |
| X | BATTISTON M. ET AL.: "Optimal framework for quantitative magnetization transfer imaging of small structures", INTERNATIONAL SOCIETY FOR MAGNETIC RESONANCE IN MEDICINE, ISMRM, 2030 ADDISON STREET, 7TH FLOOR, BERKELEY, CA 94704 USA, no. 473, 7 April 2017 (2017-04-07), XP040688041, | 1,2,5-7, 10,12-14 | **TECHNICAL FIELDS SEARCHED (IPC)** <br><br> G01R A61B G06N |
| A | * the whole document * | 3,4,8,9, 11 | |
| X | US 2017/156630 A1 (GABR REFAAT E [US] ET AL) 8 June 2017 (2017-06-08) | 1,2,5-7, 9,10, 12-14 | |
| A | * paragraph [0005]; figures 2,3 * <br> * paragraph [0005] - paragraph [0018] * <br> * paragraph [0029] - paragraph [0037] * <br> * paragraph [0043] - paragraph [0044] * | 3,4,8,11 | |

-/--

The present search report has been drawn up for all claims

| Place of search | Date of completion of the search | Examiner |
|---|---|---|
| Munich | 1 July 2022 | Lebar, Andrija |

EPO FORM 1503 03.82 (P04C01)

Europäisches
Patentamt

European
Patent Office

Office européen
des brevets

**EUROPEAN SEARCH REPORT**

Application Number

EP 22 15 6238

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2015/071514 A1 (WANG JINGHUA [US] ET AL) 12 March 2015 (2015-03-12)<br>* paragraph [0003] – paragraph [0016] *<br>----- | 1-7,10,<br>12-14<br>8,9,11 | |
| | | | TECHNICAL FIELDS SEARCHED (IPC) |

The present search report has been drawn up for all claims

| Place of search | Date of completion of the search | Examiner |
|---|---|---|
| Munich | 1 July 2022 | Lebar, Andrija |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

**EP 22 15 6238**

This annex lists the patent family members relating to the patent documents cited in the above-mentioned European search report.
The members are as contained in the European Patent Office EDP file on
The European Patent Office is in no way liable for these particulars which are merely given for the purpose of information.

01-07-2022

| Patent document cited in search report | | Publication date | Patent family member(s) | | Publication date |
|---|---|---|---|---|---|
| US 2008197841 | A1 | 21-08-2008 | NONE | | |
| US 2017156630 | A1 | 08-06-2017 | NONE | | |
| US 2015071514 | A1 | 12-03-2015 | US | 2015071514 A1 | 12-03-2015 |
| | | | US | 2016313428 A1 | 27-10-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LIN et al.** *J. Magn. Reson. Imaging,* 2021, vol. 53, 1015-1028